# EUROPEAN PATENT APPLICATION

(11) **EP 2 526 863 A1**
(43) Date of publication of application: **28.11.2012**
(21) Application number: 12168808.9
(22) Date of filing: 22.05.2012
(51) Int. Cl.: A61B 5/055, G01R 33/48

(54) **Texture stimulus presenting apparatus, magnetic resonance imaging apparatus and magnetoencephalograph including the same, and brain function measuring method**

(30) Priority: 24.05.2011 JP 2011115465; 23.11.2011 JP 2011255614
(71) Applicant: Canon Kabushiki Kaisha, Tokyo 146-8501 (JP)
(72) Inventor: Kawashima, Yasuhiro, Tokyo, Tokyo 146-8501 (JP)
(74) Representative: TBK

(57) **Abstract**

Provided is a texture stimulus presenting apparatus to be used when measuring a texture perception of a subject by detecting a change of a magnetic field in a brain of the subject. The texture stimulus presenting apparatus includes a light source configured to irradiate with light a specimen to be presented to the subject, and a control unit configured to control at least one of position of the light source, tilt of the light source, property of irradiation light from the light source, timing of irradiation, or surface morphology of the specimen facing the light source.

## Description

### BACKGROUND OF THE INVENTION

### Field of the Invention

The present invention relates to a texture stimulus presenting apparatus, a magnetic resonance imaging apparatus and a magnetoencephalograph including the texture stimulus presenting apparatus, and a brain function measuring method.

### Description of the Related Art

Human beings can learn material and characteristics of an object by perceiving form, color, and texture of the object. Types of the texture include glossiness, transparency, metallic feeling, and handling. It is known that the texture is determined by a surface state of an object and a lighting environment around the object, and the texture is one of the key properties that represent the characteristics of the object.

In recent years, lots of works have been proceeding with the texture, among which, regarding the texture representation, a considerably real image representation has become available with a development of a computer graphic technology. Conversely, not much is known about processes of how human beings perceive the texture and how texture information is processed in a brain.

An art of brain function measurement is used to study an information processing process in the brain. A magnetic resonance imaging (MRI) apparatus and a magnetoencephalograph (MEG) are representative apparatus for measuring a brain function by detecting a change of a magnetic field in the brain. The MRI apparatus applies a static magnetic field to a measurement site of a subject and further applies a specific type of high frequency magnetic field to the measurement site, to thereby generate a nuclear magnetic resonance phenomenon. The MRI apparatus obtains an image by using the nuclear magnetic resonance phenomenon thus generated.

A technique for measuring the brain function by using the MRI apparatus includes a functional MRI (fMRI) method. The fMRI method is a method of evaluating the brain function of the subject by applying various stimuli on senses such as visual, tactile, and auditory or a task such as a calculation to a subject and measuring a change of cerebral activity of the subject induced by the stimuli or the task (specifically, a change of the magnetic field associated with increase or decrease of cerebral blood flow).

However, there are several limitations in the fMRI method. The fMRI method employs measuring a considerably weak change of the magnetic field in the brain of the subject. Therefore, the MRI apparatus is installed in a magnetically shielded room, and it is required to prevent any item including magnetic material or any item possibly generating an electromagnetic noise from entering the shielded room. Further, since the fMRI method is generally performed in a dark space in the MRI apparatus, an environment of the subject during measurement is not always the same as a lighting environment in which the subject senses a texture in everyday life. Therefore, in order to measure the cerebral activity when the subject perceives the texture with the fMRI method in an accurate manner, it is desired to achieve a stimulus presenting method with no influence on the magnetic field and a technology for controlling a lighting environment when the subject perceives the texture.

Conventionally, regarding the fMRI method described above, a brain function measuring system described in Japanese Patent Application Laid-Open No. 2004-160086 has been proposed. In this system, a visual stimulus image projecting apparatus is installed in a control room in which a control device is installed, being provided adjacent to a shielded room that is electromagnetically shielded in which a main body of an MRI apparatus is installed. At the same time, a display apparatus for displaying a projected visual stimulus image is installed in the shielded room, and an opening for passing a projection light beam is provided on a boundary wall between the control room and the shielded room. That is, an image from the projecting apparatus passes through the opening and then it is displayed on the display apparatus.

As a technique for a visual environment control for the subject in an MRI measurement, a magnetic field generating apparatus for a magnetic resonance imaging apparatus described in Japanese Patent Application Laid-Open No. 2006-110043 has been proposed. This apparatus includes a light source for illuminating a space for imaging a tomographic image of a subject and a first light adjustment controller disposed in the space with which the subject in the space can adjust at least an amount of light from the light source entering into the space. That is, the subject can control the lighting environment.

### SUMMARY OF THE INVENTION

However, there are still some problems in the conventional technologies described above.

In the brain function measuring system described in Japanese Patent Application Laid-Open No. 2004-160086, the visual stimulus presented to the subject by the projecting apparatus and the display apparatus is not a real of a specimen but an image of a specimen, and hence the texture of the specimen is not reproduced perfectly. Further, since the visual stimulus to be presented is an image, a light source, which exerts large influences on a presented texture, is not included in a system configuration. Given this situation, it is difficult to implement the fMRI method to reproduce the intrinsic texture of the specimen to be presented to the subject in an accurate manner.

In the magnetic field generating apparatus described in Japanese Patent Application Laid-Open No. 2006-110043, although a light source for illuminating the space is disclosed, a target to be irradiated with light from the light source is not a specimen for presenting the texture. In addition, although it is necessary to control a positional relation between the specimen and the light source in order to present the texture of the specimen to the subject, the light source described in Japanese Patent Application Laid-Open No. 2006-110043 is disposed in the magnetic field generating apparatus, and therefore, it is difficult to perform a position control of the light source.

The present invention has been made in view of the above-mentioned problems, and it is therefore directed to a texture stimulus presenting apparatus, a magnetic resonance imaging apparatus and a magnetoencephalograph including the texture stimulus presenting apparatus, and a brain function measuring method, which enable, when measuring a brain function of a subject by detecting a change of a magnetic field in a brain, an easy position control of a light source and an easy property control of irradiation light, thus enabling an enhancement of accuracy in measuring the texture perception of the subject.

According to an exemplary embodiment of the present invention, there is provided a texture stimulus presenting apparatus used in presenting a specimen for measuring a texture perception of a subject when implementing an apparatus for measuring a brain function of the subject by detecting a change of a magnetic field, the texture stimulus presenting apparatus including: a light source configured to irradiate the specimen with light; and a control unit configured to control at least one of position of the light source, tilt of the light source, property of irradiation light from the light source, timing of irradiation, or surface morphology of the specimen facing the light source.

According to an exemplary embodiment of the present invention, there is provided a magnetic resonance imaging apparatus including the texture stimulus presenting apparatus described above.

According to an exemplary embodiment of the present invention, there is provided a magnetoencephalograph including the texture stimulus presenting apparatus described above.

Further, according to an exemplary embodiment of the present invention, there is provided a brain function measuring method, including: presenting, to a subject, a specimen for measuring a texture perception of the subject; measuring a brain function of the subject by detecting a change of a magnetic field in a brain of the subject; representing, alternately, a state in which the specimen is presented to the subject and a state in which the specimen is not presented to the subject by controlling at least one of position of a light source by which the specimen is irradiated with light, tilt of the light source, property of irradiation light from the light source, timing of irradiation, or surface morphology of the specimen facing the light source; acquiring brain function images in the state in which the specimen is presented to the subject and the state in which the specimen is not presented to the subject; and obtaining a brain function image on a cerebral activity by comparing the brain function images acquired in the state in which the specimen is presented to the subject and the state in which the specimen is not presented to the subject.

According to the present invention, when measuring the brain function of the subject by detecting a change of the magnetic field in the brain, the position control of the light source and the property control of the irradiation light can be performed in an easy manner, thus enabling an enhancement of the accuracy in measuring the texture perception of the subject.

Further features of the present invention will become apparent from the following description of exemplary embodiments with reference to the attached drawings.

### BRIEF DESCRIPTION OF THE DRAWINGS

FIG. 1 is a diagram illustrating a configuration example of a texture stimulus presenting apparatus according to an exemplary embodiment and a first embodiment of the present invention.

FIGS. 2A and 2B are enlarged diagrams of a lighting device holding unit constituting the texture stimulus presenting apparatus according to the first embodiment of the present invention.

FIG. 3 is a diagram of an example of a specimen for presenting a texture to a subject as a visual stimulus according to the first embodiment of the present invention.

FIGS. 4A, 4B, and 4C are diagrams illustrating a relation between a specimen to be presented to a subject and a light irradiation and a sequence on acquisition of a brain function image according to the first embodiment of the present invention.

FIGS. 5A and 5B are enlarged diagrams of a light intensity control device constituting a texture stimulus presenting apparatus according to a second embodiment of the present invention, which also serves as a control device constituting a texture stimulus presenting apparatus according to a third embodiment of the present invention.

FIG. 6 is an enlarged diagram of a light intensity control device constituting the texture stimulus presenting apparatus employing a linear type actuator according to the second embodiment of the present invention.

FIGS. 7A and 7B are enlarged diagrams of a control device constituting a texture stimulus presenting apparatus according to a fourth embodiment of the present invention.

FIGS. 8A and 8B are enlarged diagrams of a specimen holding unit constituting a texture stimulus presenting apparatus according to a fifth embodiment of the present invention.

FIG. 9 is an enlarged diagram of a control device constituting a texture stimulus presenting apparatus according to a sixth embodiment of the present invention.

FIG. 10 is a diagram illustrating a configuration of a magnetoencephalograph employing a texture stimulus presenting apparatus according to a seventh embodiment of the present invention.

### DESCRIPTION OF THE EMBODIMENTS

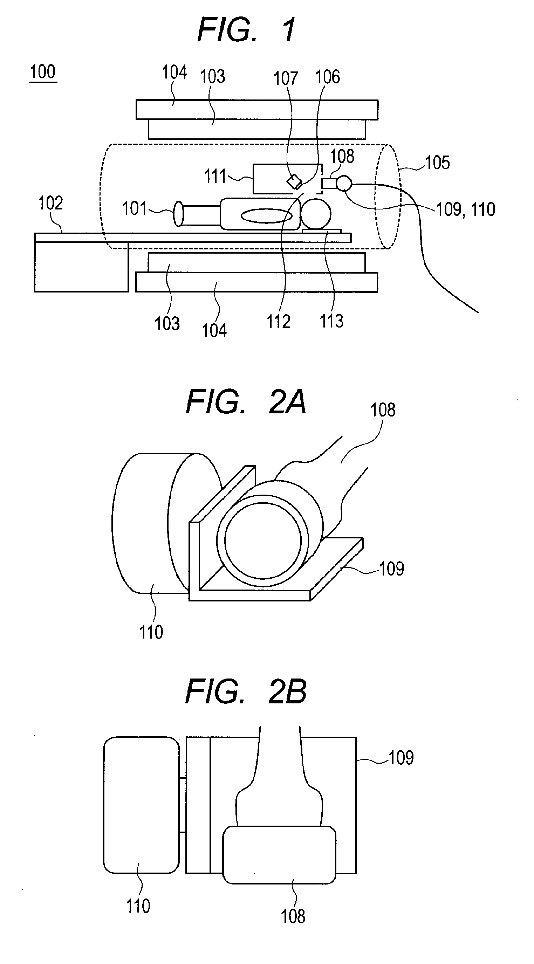

An exemplary embodiment of the present invention relates to a texture stimulus presenting apparatus for presenting a specimen for measuring a texture perception of a subject when implementing an apparatus for measuring a brain function of the subject by detecting a change of a magnetic field in a brain. For example, the texture stimulus presenting apparatus may include a light source configured to irradiate with light a specimen for measuring a texture perception of a subject and a control unit configured to control at least one of position of the light source, tilt of the light source, property of the irradiation light from the light source, timing of the irradiation, or surface morphology of the specimen facing the light source, as schematically illustrated in FIG. 1.

An MRI or an MEG is employed for the apparatus for measuring the brain function of the subject by detecting a change of the magnetic field in the brain.

The types of the texture evaluated in the brain function measurement include glossiness, transparency, and handling, and a specimen for presenting these textures to the subject as a visual stimulus can be any specimen as far as it can present a texture stimulus and it is an object formed of a nonmagnetic material. As a non-limited example, the specimen includes cloth, wood, and glass. It is preferred that the specimen for presenting the texture stimulus be set on a holding unit formed of a nonmagnetic material.

It is preferred that the control unit for selectively presenting the specimen to the subject be constituted of an actuator formed of a nonmagnetic material. It is preferred that the actuator be an ultrasonic motor. Multiple specimens for presenting the texture stimulus, multiple holding units, and multiple actuators may be provided as appropriate.

The light source according to the exemplary embodiment of the present invention can be any light source, regardless of whether it is a point light source or an area light source, as long as it can irradiate with light the specimen for presenting the texture stimulus or a visual environment space of the subject. The visual environment space means a space in which the specimen for presenting the texture stimulus exists and with which the subject has a visual contact when measuring the brain function. As a non-limited example, it is preferred that the visual environment space be a booth formed of a nonmagnetic aterial with an opening formed thereon.

Although it is best to use direct light from a light source in the light irradiation, indirect light such as light obtained by reflecting the light from the light source with a mirror can also be used.

Although it is preferred that the light source be installed at a position near the specimen for presenting the texture stimulus, the light source is not necessarily to be installed near the specimen as long as it offers a configuration for irradiating the specimen or the visual environment space with the light.

The control unit for controlling the light source according to the exemplary embodiment of the present invention can be any control unit as long as it is configured to control at least one of the position of the light source, the tilt of the light source, the property of the irradiation light from the light source, the timing of the irradiation, or the surface morphology of the specimen facing the light source. For example, it is preferred that the control unit for controlling the position or tilt of the light source be configured such that its driving can be controlled by an actuator. Among various types of actuators, it is preferred that the actuator be an ultrasonic motor formed of a nonmagnetic material. Specifically, a position control of the light source can be obtained by installing a position control unit on the light source and controlling a driving of the position control unit with a driving of the ultrasonic motor. An incident angle of light when irradiating a specimen with the light can also be controlled by applying a rotation driving of the motor as it is to a tilt control of the light source.

The property of the light that can be controlled includes amount of the irradiation light, light intensity, color temperature, chromaticity, irradiation light wavelength, and polarization of the light. For example, it is preferred to use an attenuating filter or an aperture for a light intensity control unit.

Multiple light sources and multiple control units for the light sources may be provided.

In the above-mentioned configuration, through irradiation of a specimen with light from the light source, the texture of the specimen can be presented to a subject in a brain function measurement. The texture to be presented to the subject can be controlled by a control unit controlling the position or tilt of the light source, the property of the light, the timing of the irradiation, and the surface morphology of the specimen facing the light source.

In the texture stimulus presenting apparatus according to the exemplary embodiment of the present invention, the light source described above is formed of a nonmagnetic material. When the light source is formed of a nonmagnetic material as described above, the light source can be introduced into an apparatus for performing a brain function measurement. In other words, the light source can be introduced near a specimen for presenting a texture stimulus. According to this configuration, the light source and the specimen become closer to each other, and hence an incident angle of the light on the specimen can be controlled more broadly than when the light is incident from outside the apparatus. Accordingly, various types of textures can be presented to the subject.

According to the exemplary embodiment of the present invention, a magnetic resonance imaging apparatus and a magnetoencephalograph can be built with the above-mentioned texture stimulus presenting apparatus. With such a configuration, when implementing an apparatus for measuring a brain function of a subject by detecting a change of the magnetic field in a brain, the brain function on a texture perception of the subject can be measured by using the texture stimulus presenting apparatus.

According to the exemplary embodiment of the present invention, a method of measuring a brain function of a subject when a texture of a specimen is presented to the subject can be configured as follows by using the texture stimulus presenting apparatus described above. That is, a brain function measuring method, which includes a step of representing, alternately, a state in which the specimen is presented to the subject and a state in which the specimen is not presented to the subject by controlling at least one of the position of the light source by which the specimen is irradiated with light, the tilt of the light source, the property of the irradiation light from the light source, the timing of the irradiation, or the surface morphology of the specimen facing the light source, a step of acquiring brain function images in these two states, and a step of obtaining a brain function image on a cerebral activity by comparing the brain function images acquired in the above-mentioned two states with each other, can be configured specifically as follows.

In the step of representing each of the state in which the specimen for measuring the texture perception is presented to the subject and the state in which the specimen is not presented to the subject by using the control unit when irradiating the specimen with the light from the light source, when presenting the specimen to the subject, the position of the specimen for presenting the texture, the position or tilt of the light source, and the property of the irradiation light are set constant. In this state, the specimen is irradiated with the light for a predetermined time, by which the state of presenting the specimen for measuring the texture perception can be demonstrated to the subject.

On the other hand, when the controlled state is changed, the state of not presenting the specimen for measuring the texture perception can be demonstrated to the subject. For example, it is only necessary to change the position or tilt of the light source or the property of the irradiation light by using the control unit for controlling the light source after an elapse of the predetermined time. The property of the light that can be changed includes the amount of the irradiation light, the light intensity, the color temperature, the chromaticity, the irradiation light wavelength, and the polarization of the light.

Alternatively, when the light irradiation is stopped, the state of not presenting the specimen for measuring the texture perception can also be achieved. Alternatively, when the state of presenting the specimen for presenting the texture is changed without changing the position or tilt of the light source and the property of the irradiation light, the state of not presenting the specimen for measuring the texture perception to the subject can be achieved.

In the step of acquiring the brain function image in each of the states alternately represented in the above-mentioned step, in a case where an actuator is used for a driving control in a control unit for controlling the light source or a unit for presenting the specimen for presenting the texture, it is preferred to stop acquiring the brain function image in a period in which the actuator is being driven.

In the step of obtaining the brain function image, on which the cerebral activity is reflected, by comparing the brain function images acquired in the above-mentioned step, for example, it is preferred to compare the acquired brain function images to obtain the brain function image, on which the cerebral activity is reflected, based on a statistical procedure. It is preferred that the brain function images to be compared be a brain function image in the state of presenting the specimen for measuring the texture perception and a brain function image in the state of not presenting the specimen. Through acquisition of the brain function images in the state of presenting the specimen for measuring the texture perception and the state of not presenting the specimen alternately represented to the subject and comparison of the acquired images with each other, it is possible to achieve a configuration for obtaining the brain function image on which the cerebral activity is reflected. This enables an implementation of the brain function measurement when the subject perceives the texture.

The step of representing each of the state of presenting the specimen for measuring the texture perception to the subject and the state of not presenting the specimen can be configured such that the state of presenting the specimen for measuring the texture perception and the state of not presenting the specimen are repeated, alternately, multiple times at regular intervals. With this configuration, when a presentation of the specimen for measuring the texture perception is performed multiple times in a repeated manner at regular intervals, an image processing such as an averaging can be performed on the acquired brain function images, enabling a comparison of the images with less noise.

It is preferred to take a large number of repetitions in order to improve the accuracy of the measurement. The number of repetitions can be set appropriately considering the accuracy of the measurement and a burden on the subject.

(Embodiments)

Specific embodiments of the present invention are described below.

(First Embodiment)

FIG. 1 is a diagram illustrating a configuration example of a texture stimulus presenting apparatus to which the present invention is applied.

In FIG. 1, an MRI apparatus 100 includes a bed 102 on which a subject 101 is placed, a gradient coil 103, a superconducting magnet 104, and a bore of the scanner 105. The subject 101 lies down on the bed 102 in the bore of the scanner 105, and a specimen 106 for presenting a texture to the subject 101 as a visual stimulus is provided by a specimen holding unit 107 in front of eyes of the subject 101. An optical fiber lighting device 108 for irradiating the specimen 106 with light is installed above a head of the subject 101. A lighting device holding unit 109 and an ultrasonic motor 110 are mounted to the lighting device 108, and the position and tilt of the lighting device 108 can be controlled by performing a control of the lighting device holding unit 109 with the ultrasonic motor 110.

As the ultrasonic motor 110, one of the devices configured to be driven by a piezoelectric element (electrical-mechanical energy converting element) disclosed in a number of literatures, such as Japanese Patent Application Laid-Open No. 3-253272, can be used.

The specimen 106 and the specimen holding unit 107 are contained in a booth 111 having an opening 112 through which the subject 101 can perceive the specimen 106. Irradiation light from the lighting device 108 is configured to be introduced into the booth 111.

Each of the specimen 106, the specimen holding unit 107, the lighting device 108, the lighting device holding unit 109, the ultrasonic motor 110, and the booth 111 is formed of a nonmagnetic material.

A coil 113 for detecting an MR signal is installed on the backside of the head of the subject 101, which detects an electromagnetic signal generated by a change of a cerebral blood flow accompanied by a neural activity of the subject 101. In this case, in order to secure a view in front of the subject and to perform a highly sensitive brain function measurement in a visual cortex of the brain (corresponding to the occipital region of the subject), the coil 113 to be used is a surface coil type radio frequency coil.

FIGS. 2A and 2B are enlarged diagrams of the lighting device holding unit 109 constituting the texture stimulus presenting apparatus according to the first embodiment of the present invention. The lighting device 108 is installed on a base of the lighting device holding unit 109. The ultrasonic motor 110 is mounted on a side portion of the lighting device holding unit 109. A rotation shaft of the ultrasonic motor 110 is connected to the center of the side portion of the lighting device holding unit 109 so that the lighting device holding unit 109 is rotatable around the rotation shaft.

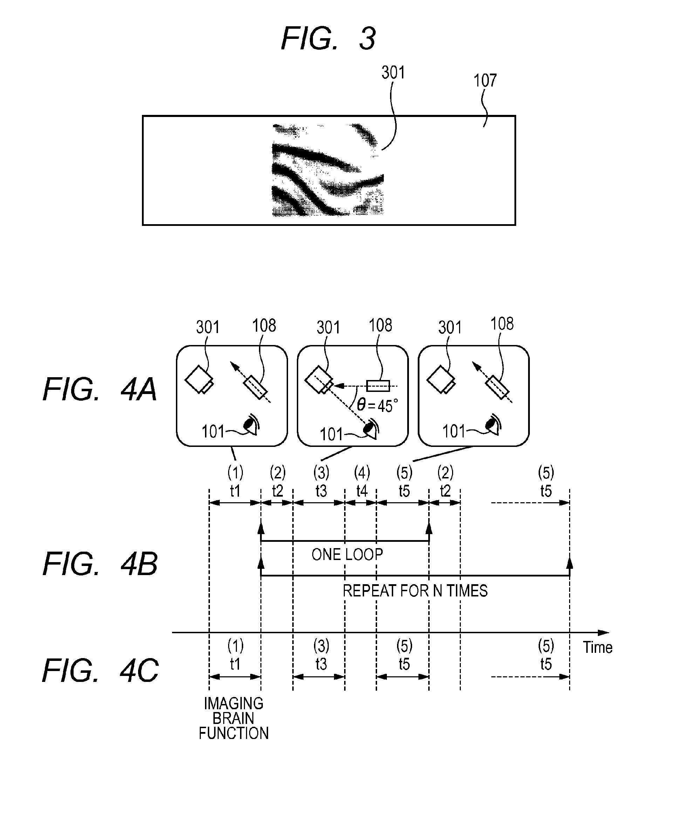

FIG. 3 is a diagram of an example of a specimen for presenting a texture to the subject as a visual stimulus in the first embodiment of the present invention. In this case, for ease of description, it is assumed that a measurement is performed using a cloth 301 illustrated in FIG. 3 as the specimen 106 for presenting the texture to the subject 101 as a visual stimulus. The cloth 301 is assumed to be set on the specimen holding unit 107. In this embodiment, no magnetic material is contained in the cloth 301.

The bed 102 is set such that the head of the subject 101 is placed in the bore of the scanner 105 of the MRI apparatus 100. The specimen holding unit 107 is set in front of eyes of the subject 101 such that the subject 101 can perceive the cloth 301. The cloth 301 to be perceived by the subject 101 is configured to be irradiated with light from the lighting device 108.

FIGS. 4A, 4B, and 4C are diagrams illustrating a relation between the specimen 106 to be presented to the subject 101 and the light irradiation and a sequence on acquisition of a brain function image according to the first embodiment. The relation between the specimen 106 to be presented to the subject 101 and the light irradiation is illustrated in FIGS. 4A and 4B.

The specimen 106 for measuring the texture perception is presented to the subject 101 in the following Steps (1) to (5). FIG. 4B illustrates an order of the steps and a time interval of each step, and FIG. 4A illustrates a temporal change among the cloth 301, the lighting device 108, and the subject 101.

Before Step (1), the position and tilt of the lighting device 108 are controlled by the ultrasonic motor 110 such that a light irradiation direction of the lighting device 108 and a direction of eyes of the subject 101 become parallel to each other. Firstly, in Step (1), the cloth 301 is presented to the subject 101 for a time interval t1 in a state in which the light irradiation direction of the lighting device 108 and the direction of eyes of the subject 101 are parallel to each other.

After that, in Step (2), the ultrasonic motor 110 is rotated during a time interval t2, by which the light irradiation direction of the lighting device 108 and the direction of eyes of the subject 101 are controlled to form an angle of 45 degrees. With this operation, an incident angle of the irradiation light on the cloth 301 is controlled to be 45 degrees.

Subsequently, in Step (3), with the irradiation position fixed, the cloth 301 is presented to the subject 101 for a time interval t3.

After that, in Step (4), the ultrasonic motor 110 is rotated in a direction opposite to a direction in Step (2) for a time interval t4 to change the tilt of the lighting device 108, by which the light irradiation direction of the lighting device 108 and the direction of eyes of the subject 101 are controlled to be parallel to each other.

Finally, in Step (5), with the irradiation position fixed, the cloth 301 is presented to the subject 101 for a time interval t5.

The above-mentioned Steps (2) to (5) make a loop, and the loop of driving the ultrasonic motor 110 and changing the tilt of the lighting device 108 is repeated N times in an order of Step (2), Step (3), Step (4), Step (5), Step (2), ..., Step (5). At this time, the property of the irradiation light, such as the amount of the light, is kept constant.

Among multiple methods for presenting the stimulus to the subject used in the brain function measurement by the fMRI method, the first embodiment employs a method called "block design" that is used in a number of brain function measurements. In the block design method, a state in which the specimen for measuring the texture perception is not presented to the subject 101, which is called a "rest block", and a state in which the specimen for measuring the texture perception is presented to the subject 101, which is called a "task block", are repeated alternately. In the first embodiment, Step (3) corresponds to the task block and Step (5) corresponds to the rest block. In Step (5), although the cloth 301 is presented to the subject 101, since the cloth 301 is not irradiated with the light, the subject 101 cannot perceive the cloth 301. That is, in Step (5), the cloth 301 is not the texture stimulus to the subject 101. Therefore, Step (5) is in a state where the specimen 106 for measuring the texture perception is not presented to the subject 101, and Step (5) corresponds to the rest block.

As illustrated in FIG. 4C, a timing for presenting the specimen 106 for measuring the texture perception to the subject 101 and a timing for starting imaging of the fMRI method are synchronized with each other. In the task block, i.e., during the time interval t3 of Step (3), a brain function image when the cloth 301 is presented to the subject 101 is acquired, and in the rest block, i.e., during the time interval t5 of Step (5), a brain function image when the cloth 301 is not presented to the subject 101 is acquired. These images are two types of images including a brain function image in the state in which the specimen for measuring the texture perception is presented to the subject and a brain function image in the state in which the specimen is not presented to the subject. In the first embodiment, the loop from Step (2) to Step (5) is repeated N times, and hence N brain function images are acquired in each of the task block and the rest block.

A noise is removed from the images by performing an image processing such as an averaging, and finally by comparing the brain function images in the task block and the rest block, a brain function image when the specimen for measuring the texture perception is presented to the subject can be obtained. Through an analysis of the obtained brain function image, the brain function when the subject perceives the texture can be measured.

In the brain function image acquiring sequence according to the first embodiment, the specimen 106 presented to the subject 101 is changed by rotating the ultrasonic motor 110 in Steps (2) and (4). It is preferred to create the brain function image acquiring sequence of the fMRI method so as not to acquire the brain function images during the time intervals t2 and t4.

One of the reasons why it is preferred to create the brain function image acquiring sequence of the fMRI method so as not to acquire the brain function images during the time intervals t2 and t4 is that the texture stimulus presenting apparatus according to the first embodiment changes the tilt of the light source during the time intervals t2 and t4, and hence there is a possibility that both images in the task block and the rest block exist in a mixed manner in the brain function images acquired during these time intervals. Another reason is that there is a possibility that a weak electromagnetic wave generated from driving of the ultrasonic motor 110 causes a degradation of the brain function images.

When the acquisition of the brain function images is stopped during the time intervals for driving the ultrasonic motor 110, a noise caused by the driving of the ultrasonic motor 110 can be suppressed from being superimposed on image data to be used in an analysis after the measurement.

Depending on the type of the MRI apparatus, some MRI apparatus cannot stop the acquisition of the brain function images only during the time intervals t2 and t4 as described above. In such cases, a desired brain function image can be obtained by analyzing only the image data acquired during the time intervals t3 and t5 without using the brain function images acquired during the time intervals t2 and t4 after acquiring all the brain function images across the whole measurement period.

The present invention is not limited to a configuration in which the brain function measurement is performed by presenting the specimen for measuring the texture perception to the subject by using the texture stimulus presenting apparatus as described in the first embodiment, and the lighting device is not limited to the optical fiber lighting device formed of a nonmagnetic material.

Although a front surface of the specimen to be presented to the subject is irradiated with the light in the first embodiment, a texture on the light transmission property can also be presented to the subject by irradiating with the light a back surface of a specimen that is light-transmissive. Not only with the light irradiation within the MRI apparatus, but also with direct light from a light source outside the MRI apparatus, such as a projector, or indirect light obtained by reflecting light from a light source with a mirror, the specimen can be irradiated. Further, multiple light sources and multiple control units for the light sources may be provided as appropriate.

The control of the position and tilt of the lighting device in the brain function image acquiring sequence is not limited to that in the brain function image acquiring sequence according to the first embodiment as long as the position and tilt of the lighting device are set to irradiate with light the specimen 106 to be presented to the subject 101.

Although the property of the light is kept constant in the first embodiment, the brain function measurement can also be performed by using the texture stimulus presenting apparatus while controlling the property of the light. In this case, the property of the light that can be controlled includes properties such as the light intensity, the color temperature, the chromaticity, the light wavelength, and the polarization of the light. Further, the brain function measurement can also be performed by using the texture stimulus presenting apparatus while controlling on and off of the light irradiation.

Although the cloth is used as the specimen for presenting the texture to the subject as the visual stimulus in the first embodiment, wood and glass can also be used but not limited to these materials as long as textures can be presented to the subject and the material is nonmagnetic.

(Second Embodiment)

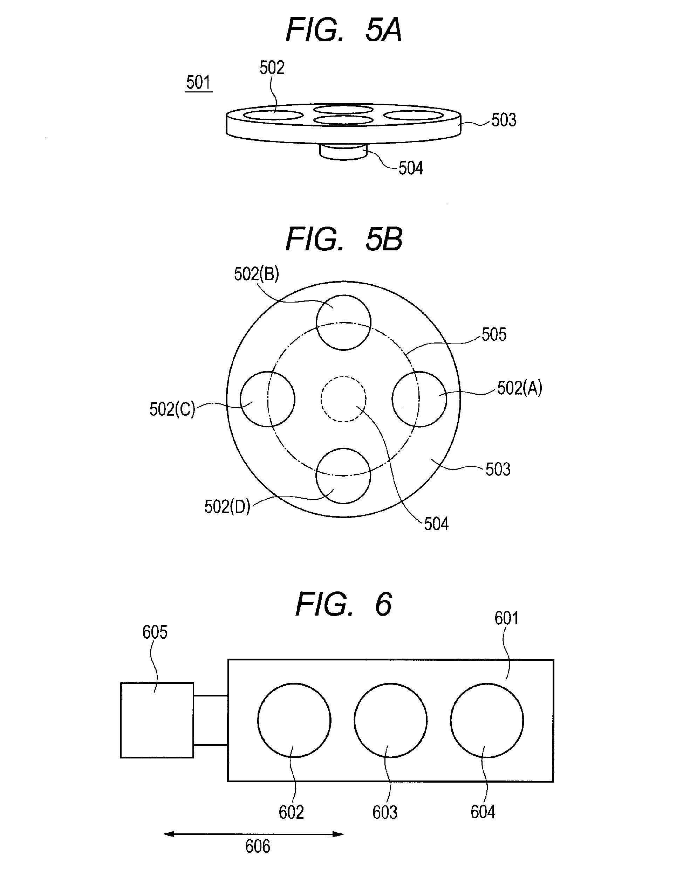

FIGS. 5A and 5B are enlarged diagrams of a light intensity control device 501 in a configuration example including a light intensity control unit to control the light intensity of the light source constituting the texture stimulus presenting apparatus. The light intensity control device 501 is disposed between the specimen 106 and the lighting device 108 illustrated in FIG. 1. FIG. 5A is a perspective projection view of the light intensity control device 501, and FIG. 5B is a plan view of the light intensity control device 501. As illustrated in FIGS. 5A and 5B, multiple light intensity control units 502 are disposed on a holding unit 503.

An ultrasonic motor 504 is mounted to a back surface of the holding unit 503. The rotation shaft of the ultrasonic motor 504 is connected to the center of the holding unit 503, and the holding unit 503 is rotatable around this rotation shaft. The light intensity control units 502, the holding unit 503, and the ultrasonic motor 504 are formed of nonmagnetic material.

A procedure for selectively and continuously changing the light intensity control unit according to the second embodiment of the present invention is described below. In this description, the intensity of the light with which the specimen is irradiated is controlled by an attenuating filter used as the light intensity control unit 502. With the use of the attenuating filter, the light intensity can be controlled with a minimal change of the property of the light other than the light intensity, such as the color temperature and the chromaticity.

Specifically, as illustrated in FIG. 5B, attenuating filters 502(A), 502(B), 502(C), and 502(D) each having different transmissivity are disposed. The attenuating filters 502(A) to 502(D) contain no magnetic material.

The light intensity control device 501 is disposed such that a trajectory 505 drawn by the driving of the ultrasonic motor 504 passes a line connecting the specimen 106 and the lighting device 108, i.e., an optical path of the light. The specimen 106 can be irradiated with the light in a state in which the light intensity is controlled by rotating the ultrasonic motor 504 and fixing a desired one of the attenuating filters 502(A) to 502(D) each having different transmissivity on the optical path of the light.

In the second embodiment, a cloth is used as the specimen for presenting the texture to the subject as the visual stimulus, in the same manner as in the first embodiment, to perform the measurement.

With the configuration according to the second embodiment described above, the brain function measurement can be performed by controlling the intensity of the light with which the specimen for presenting the texture is irradiated. With the configuration in which the texture stimulus presenting apparatus as described in the first embodiment is used in combination, the texture can be presented to the subject in more parametric manner.

Although the attenuating filter is used as the light intensity control unit in the second embodiment, the light intensity control unit is not limited to the control unit as described above as long as it is a unit that can control the light intensity. For example, apertures having different aperture diameters can also be used as the light intensity control unit.

As a driving unit for the light intensity control device 501, not only a rotation type actuator driven by an ultrasonic motor as described in the second embodiment, but also a linear type actuator can be used. When implementing the linear type actuator, as illustrated in FIG. 6, multiple light intensity control units (602, 603, and 604) are set in a line on a holding unit 601, and a linear type actuator 605 is connected to the holding unit 601. The linear type actuator 605 is configured to move the holding unit 601 in a linear direction indicated by a double-headed arrow 606.

(Third Embodiment)

With the same configuration as the light intensity control unit illustrated in FIGS. 5A and 5B, the light intensity control unit can be used as a control unit 502 for controlling the property of the irradiation light from the light source constituting the texture stimulus presenting apparatus.

In the third embodiment of the present invention, the polarization of the light with which the specimen is irradiated is controlled by selectively and continuously changing the control unit by using a polarizing plate as the control unit 502. Specifically, as illustrated in FIG. 5B, polarizing plates 502(A), 502(B), 502(C), and 502(D) each having a different polarization direction are disposed. The polarizing plates 502(A) to 502(D) contain no magnetic material.

The light intensity control device 501 is disposed such that the trajectory 505 drawn by the driving of the ultrasonic motor 504 passes a line connecting the specimen 106 and the lighting device 108, i.e., an optical path of the light.

A procedure of a brain function measuring method by the fMRI method according to the third embodiment is similar to that in the second embodiment. The specimen can be irradiated with the light in a state in which the property of the light is controlled by rotating the ultrasonic motor 504 and fixing a desired one of the polarizing plates 502(A) to 502(D) each having a different polarization direction on the optical path of the light.

With the configuration according to the third embodiment described above, the brain function measurement can be performed by controlling the polarization direction of the light with which the specimen for presenting the texture is irradiated. With the configuration in which the texture stimulus presenting apparatus as described in the first embodiment is used in combination, the texture can be presented to the subject in more parametric manner. Although the polarizing plate is used as the control unit in the third embodiment, besides this, a color filter configured to control the chromaticity, a diffusing plate configured to control a diffusion state of the light, or the like can be used as the control unit. In this manner, the control unit is not limited to the control unit as described above as long as it is a unit that can control the property of the light.

(Fourth Embodiment)

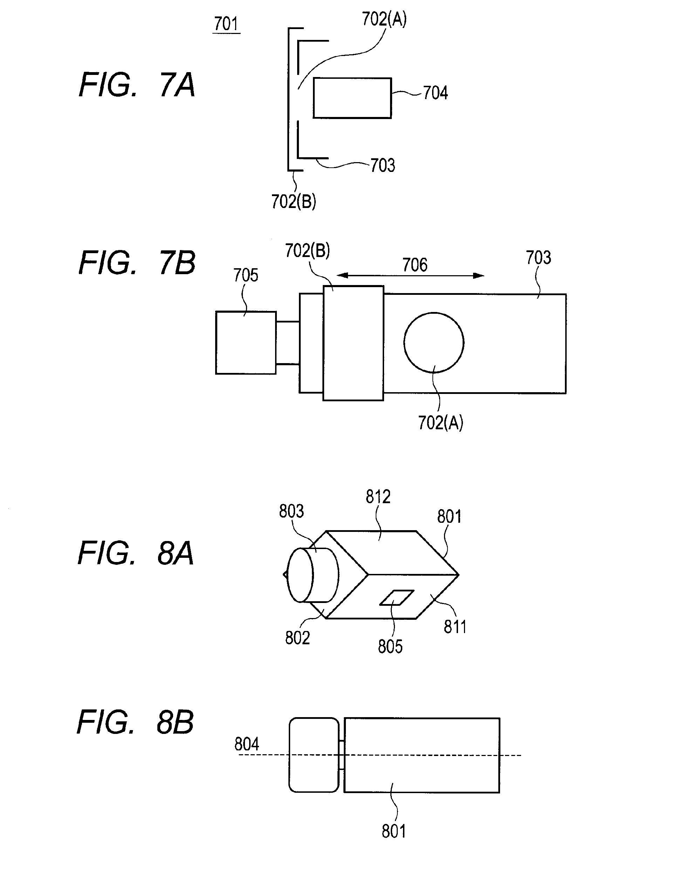

FIG. 7B is an enlarged diagram of a control device 701 for controlling a timing of the light irradiation from the light source constituting the texture stimulus presenting apparatus. Control units 702(A) and 702(B) are disposed on a holding unit 703. The control unit 702(A) is an aperture for allowing the light to pass, and the control unit 702(B) serves as a lid for covering the aperture. Further, as illustrated in FIG. 7A, the control device 701 is disposed around a light irradiation portion of a lighting device 704. A linear type actuator 705 is mounted to the holding unit 703, which is configured to move the control unit 702(B) in a linear direction indicated by a double-headed arrow 706. The control unit 702, the holding unit 703, the lighting device 704, and the linear type actuator 705 are formed of nonmagnetic material.

With the configuration according to the fourth embodiment of the present invention described above, the brain function measurement can be performed by controlling the timing of the light irradiation of the specimen for presenting the texture. With the configuration in which the texture stimulus presenting apparatus as described in the first embodiment is used in combination, the texture can be presented to the subject in an intermittent manner.

(Fifth Embodiment)

FIGS. 8A and 8B are enlarged diagrams illustrating a configuration of a texture stimulus presenting apparatus including a specimen holding unit 801 with an ultrasonic motor. The specimen holding unit 801 is formed of a nonmagnetic material in a cuboid, and the specimen to be presented to the subject is set on a surface from among four rectangular surfaces constituting the cuboid. An ultrasonic motor 803 formed of a nonmagnetic material is mounted to an end surface portion 802 of a square constituting the cuboid. A rotation shaft 804 of the ultrasonic motor 803 is connected to the center of the end surface portion 802 so that the specimen holding unit 801 is rotatable around the rotation shaft 804.

A controller (not shown) that controls driving of the ultrasonic motor 803 is disposed in a magnetically shielded room in which the MRI apparatus is installed, at a place having the maximum distance from a measurement position in a main unit of the MRI apparatus. The controller is then connected to the ultrasonic motor 803 with a control line that is electromagnetically shielded.

In this manner, the ultrasonic motor 803 is configured such that the time and the rectangular surface for presenting the specimen can be controlled through the driving by the controller when presenting the specimen to the subject in a selective manner.

In the brain function measuring method by the fMRI method according to the fifth embodiment of the present invention, a cloth is used as the specimen for presenting the texture to the subject as the visual stimulus, in the same manner as in the first embodiment, to perform the measurement. A cloth 805 is set on paper having a whole surface evenly colored with gray, being attached on a surface 811 of the specimen holding unit 801. On the other hand, paper having the same gray color as the paper on which the cloth 805 is set is attached on a surface 812 of the specimen holding unit 801. In this case, the cloth 805, an ink material used in printing, and the paper contain no magnetic material. In front of eyes of the subject, the specimen holding unit 801 is set at a position where the subject can perceive the cloth 805 attached on the surface 811. At the same time, surfaces of the specimen holding unit 801 other than the surface 811 are set at positions that cannot be perceived by the subject. The cloth 805 to be perceived by the subject is configured to be irradiated with light from a lighting device.

With the configuration according to the fifth embodiment, the brain function measurement can be performed by switching the specimen for presenting the texture to the subject. With the configuration in which the texture stimulus presenting apparatus as described in the first embodiment is used in combination, the texture can be presented to the subject in more parametric manner.

Multiple ultrasonic motors may be provided as appropriate.

(Sixth Embodiment)

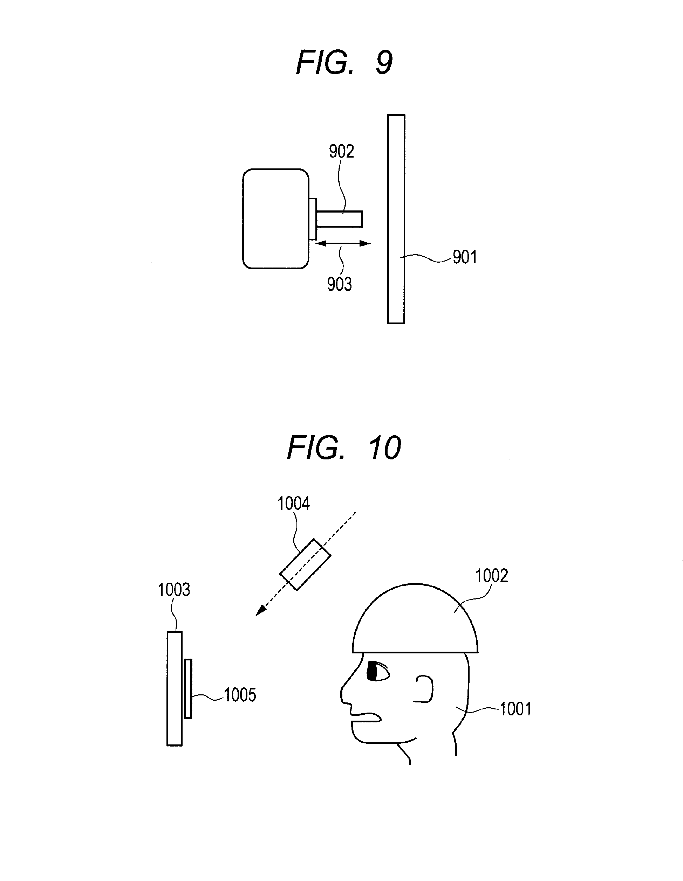

FIG. 9 is a diagram illustrating a configuration example of a texture stimulus presenting apparatus including a control unit for controlling a form of a specimen. Through the control of the form of the specimen, a light reflection direction obtained when light from a light source is reflected at a surface of the specimen can be controlled. As illustrated in FIG. 9, a linear type actuator 902 is disposed on a back surface side of a specimen 901, i.e., a surface opposite to a surface to be presented to the subject. A tip of the actuator is then moved in a linear direction indicated by a double-headed arrow 903 to press the back surface of the specimen 901, by which the form of the specimen 901 can be changed. That is, the light reflection direction obtained when the light from the light source is reflected at the surface of the specimen 901 can be controlled.

With the configuration according to the sixth embodiment of the present invention, the form of the specimen for presenting the texture to the subject can be controlled. That is, the brain function measurement can be performed by controlling the light reflection direction obtained when the light from the light source is reflected at the surface of the specimen. With the configuration in which the texture stimulus presenting apparatus as described in the first embodiment is used in combination, the texture can be presented to the subject in more parametric manner.

Although the linear type actuator is used as the control unit in the sixth embodiment, the control unit is not limited to the control unit as described above as long as it is a unit that can control the form of the specimen.

(Seventh Embodiment)

FIG. 10 is a diagram illustrating a configuration example of a magnetoencephalograph (MEG) employing a texture stimulus presenting apparatus according to the present invention. When this type of magnetoencephalograph is used, a change of the cerebral activity can be measured and obtained as an image with an excellent temporal accuracy. A main body of the magnetoencephalograph (not shown) and the texture stimulus presenting apparatus are installed in a magnetically shielded room. A subject 1001 wears a helmet-shaped dewar type sensor assembly 1002 on his/her head. The sensor assembly 1002 has a magnetic sensor contained inside. A specimen holding unit 1003 of the texture stimulus presenting apparatus is disposed in front of eyes of the subject 1001. A lighting device 1004 is installed such that a specimen 1005 for presenting the texture stimulus set on the specimen holding unit 1003 is irradiated with light from the lighting device 1004.

A procedure of the brain function measurement using this system is similar to the procedures of the fMRI method described in the first to sixth embodiments.

As in the seventh embodiment of the present invention, when the magnetoencephalograph is configured using the texture stimulus presenting apparatus according to the present invention, the brain function measurement can be performed with less noise applied to the magnetoencephalograph.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.
Provided is a texture stimulus presenting apparatus to be used when measuring a texture perception of a subject by detecting a change of a magnetic field in a brain of the subject. The texture stimulus presenting apparatus includes a light source configured to irradiate with light a specimen to be presented to the subject, and a control unit configured to control at least one of position of the light source, tilt of the light source, property of irradiation light from the light source, timing of irradiation, or surface morphology of the specimen facing the light source.

## Claims

1. A texture stimulus presenting apparatus used in presenting a specimen for measuring a texture perception of a subject when implementing an apparatus for measuring a brain function of the subject by detecting a change of a magnetic field, the texture stimulus presenting apparatus comprising:
a light source configured to irradiate the specimen with light; and
a control unit configured to control at least one of position of the light source, tilt of the light source, property of irradiation light from the light source, timing of irradiation, or surface morphology of the specimen facing the light source.

2. The texture stimulus presenting apparatus according to claim 1, wherein the light source is formed of a nonmagnetic material.

3. The texture stimulus presenting apparatus according to claim 1, wherein the control unit comprises an actuator formed of a nonmagnetic material.

4. The texture stimulus presenting apparatus according to claim 3, wherein the actuator comprises an ultrasonic motor.

5. A magnetic resonance imaging apparatus, comprising the texture stimulus presenting apparatus according to claim 1.

6. A magnetoencephalograph, comprising the texture stimulus presenting apparatus according to claim 1.

7. A brain function measuring method, comprising:
presenting, to a subject, a specimen for measuring a texture perception of the subject;
measuring a brain function of the subject by detecting a change of a magnetic field in a brain of the subject;
representing, alternately, a state in which the specimen is presented to the subject and a state in which the specimen is not presented to the subject by controlling at least one of position of a light source by which the specimen is irradiated with light, tilt of the light source, property of irradiation light from the light source, timing of irradiation, or surface morphology of the specimen facing the light source;
acquiring brain function images in the state in which the specimen is presented to the subject and the state in which the specimen is not presented to the subject; and
obtaining a brain function image on a cerebral activity by comparing the brain function images acquired in the state in which the specimen is presented to the subject and the state in which the specimen is not presented to the subject.

8. The brain function measuring method according to claim 7, wherein the representing comprises repeating the state in which the specimen is presented to the subject and the state in which the specimen is not presented to the subject multiple times at regular intervals, alternately.
